# EUROPEAN PATENT APPLICATION

(11) **EP 3 235 490 A1**
(43) Date of publication of application: **25.10.2017**
(21) Application number: 16305451.3
(22) Date of filing: 18.04.2016
(51) Int. Cl.: A61K 8/27, A61K 8/46, A61Q 5/00, A61K 8/67, A61K 8/97

(54) **COMPOSITION FOR IMPROVING HAIR QUALITY**

(71) Applicant: L'Oréal, 75008 Paris (FR); Nestec S.A., 1800 Vevey (CH)
(72) Inventor: PICCARDI, Nathalie, 21310 Arceau (FR); CONNETABLE-MCMAHON, Sophie, 93601-Aulnay-Sous-Bois (FR); DEMESSANT-FLAVIGNY, Ann'Laure, 92110 Clichy (FR)
(74) Representative: Nony

(57) **Abstract**

The present invention relates to a combination comprising grape seed extract, vitamin C, zinc, taurine and biotin.

The present invention also relates to a composition comprising the combination of the invention, which is particularly useful for improving the quality of hair and/or reducing the emotional impact of thin hair.

## Description

Thin hair have a considerable emotional impact, in particular on women.

The present invention is directed to a composition for improving the quality of hair, in particular their thickness, their strength, their manageability, their volume, their shininess, and/or their appearance.

The present invention is also directed to a composition for reducing the emotional impact of thin hair.

According to one aspect, the present invention is directed to a combination comprising grape seed extract, vitamin C, zinc, taurine and biotin.

Preferably, the combination consists of grape seed extract, vitamin C, zinc, taurine and biotin.

According to another aspect, the present invention is directed a composition comprising 187 mg of grape seed extract, 30 mg of vitamin C, 7 mg of zinc, 150 mg of taurine, and 30 µg of biotin.

Preferably, the composition of the invention also comprises an excipient suitable for oral administration.

Preferably, the composition of the invention is a food supplement.

Preferably, the composition of the invention is a tablet.

According to another aspect, the present invention is directed to the use of a composition comprising the combination of the invention, for improving the quality of hair.

Preferably, the composition comprises 187 mg of grape seed extract, 30 mg of vitamin C, 7 mg of zinc, 150 mg of taurine, and 30 µg of biotin.

The use of the invention is in particular for improving the thickness, the strength, the manageability, the volume, the shininess, and/or the appearance of hair.

The use of the invention is in particular for reducing the emotional impact of thin hair, in particular on women.

According to another aspect, the present invention is directed to a method for improving the quality of hair comprising the oral administration of a composition according to the invention.

The administration is preferably daily.

According to another aspect, the present invention is directed to a method for reducing the emotional impact of thin hair comprising the oral administration of a composition according to the invention.

The administration is preferably daily.

### MATERIALS & METHODS

An observational study was performed at 16 private dermatology practices in Poland (June to October 2014) and enrolled 113 healthy Caucasian females (mean 40.1 years; range 18-60) with thin hair.

After baseline assessments, subjects took 2 tablets of the food supplement according to the invention daily for 3 months (grape seed extract 187mg, vitamin C 30mg, zinc 7mg, taurine 150mg, biotin 30µg) and received the same mild shampoo.

Subjects self-rated agreement with statements about hair quality (eg thickness, strength, manageability and appearance) on a 5-point scale. Dermatologists and hair professionals assessed hair quality on a 10-point scale. Subjects answered 14 questions about emotional impact (eg relationships, depression, anxiety and appearance and activity) on a 5-point scale (total possible score 42). Subjects and dermatologists evaluated hair quality and emotional impact at baseline and days 15 (self assessment only), 30, 60 and 90. Hair professionals evaluated hair quality at baseline and day 90. Statistical significance (dermatologists'/ hair professionals' evaluations and emotional impact composite score) was assessed using Student's t-test.

### RESULTS

Improved hair quality and reduced emotional impact compared to baseline was apparent after day 15 based on self-assessment and 1 month based on dermatologist assessment and emotional impact score. Outcomes further improved during follow up.

After 3 months:
- 78% of subjects agreed or strongly agreed with "My hair is stronger";
- 73% with "My hair is less breakable"; and
- 68% with "My hair is thicker".

In addition:
- 81% agreed or strongly agreed with "My hair is easier to style";
- 79% with "My hair volume has improved";
- 75% with "My hair is shinier";
- 74% with "My hair is easier to untangle", "My hair is softer to touch" and "I have less flyway (electric) hair";
- 72% with "My hair is less dry";
- 65% with "My hairstyle lasts longer between washes"; and
- 63% with "My scalp is less visible".

Compared to baseline, after 3 months, dermatologists' scores for weakness / brittleness (mean 3.6v1.6 respectively), thin hair (5.8v4.7), bounce (3.5v6.1) volume and density (both 3.6v5.9) and scalp visibility (4.2v2.5) significantly (all p<0.01) improved.

Composite emotional impact score declined compared to baseline after 3 months (mean 21.9v18.2 respectively p<0.01) as did hair professionals' scores for thickness (3.5v5.0), brittleness (3.9v2.3), dry hair (4.3v2.5), fragility (4.9v3.3), manageability (4.1v5.9), weakness (4.4v2.5) and 'electrified' hair (3.6v2.0) significantly improved (all p<0.01). The supplement was well tolerated.

### CONCLUSION

In women with thin hair, the food supplement improved hair thickness, strength, manageability, appearance and other aspects of quality, and reduced emotional impact.

## Claims

1. A combination comprising grape seed extract, vitamin C, zinc, taurine and biotin.

2. A composition comprising 187 mg of grape seed extract, 30 mg of vitamin C, 7 mg of zinc, 150 mg of taurine, and 30 µg of biotin.

3. The composition according to claim 2, **characterized in that** it is a food supplement.

4. The composition according to claim 2 or 3, **characterized in that** it is a tablet.

5. Use of a composition comprising the combination of claim 1 for improving the quality of hair.

6. The use according to claim 5, wherein the composition is according to any one of claims 2 to 4.

7. The use according to claim 5 or 6, for improving the thickness, the strength, the manageability, the volume, the shininess, and/or the appearance of hair.

8. The use according to claim 5 or 6, for reducing the emotional impact of thin hair.

9. Method for improving the quality of hair comprising the oral administration of a composition according to any one of claims 2 to 4.

10. Method according to claim 9 wherein the administration is daily.

11. Method for reducing the emotional impact of thin hair comprising the oral administration of a composition according to any one of claims 2 to 4.

12. Method according to claim 11 wherein the administration is daily.
